# EUROPEAN PATENT APPLICATION

(11) **EP 3 622 901 A1**
(43) Date of publication of application: **18.03.2020**
(21) Application number: 19196722.3
(22) Date of filing: 11.09.2019
(51) Int. Cl.: A61B 17/12

(54) **ANEURYSM OCCLUSION DEVICE**

(30) Priority: 12.09.2018 US 201816128929
(71) Applicant: DePuy Synthes Products, Inc., Raynham, MA 02767 (US)
(72) Inventor: The designation of the inventor has not yet been filed
(74) Representative: Small, Gary James

(57) **Abstract**

An occlusion device suitable for endovasculature treatment of an aneurysm in a blood vessel in a patient, including a substantially tubular structure having a proximal end region and a distal end region, having a first, expanded condition and a second, collapsed condition. The device has dimensions in the second, collapsed condition suitable for insertion through vasculature of the patient and through a neck of the aneurysm. The device further includes a control ring having a substantially annular body disposed on the proximal end region of the structure and at least substantially circumscribing the proximal end region to prevent radial expansion of the proximal end region and to provide an engagement feature during manipulation of the occlusion device.

## Description

### CROSS-REFERENCE TO RELATED APPLICATIONS

This application is a continuation in part of U.S. patent application serial number 14/230,426, filed on March 31, 2014, the content of which is incorporated herein by reference in its entirety.

### BACKGROUND OF THE INVENTION

### 1. Field of Invention

The invention relates to implants within body vessels and more particularly to occlusion devices for small vascular openings such as a neck of an aneurysm.

### 2. Description of the Related Art

Vascular disorders and defects such as aneurysms and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

In the treatment of aneurysms by endovascular implants, the goal is to exclude the internal volume of the aneurysm sac from arterial blood pressure and flow. As long as the interior walls of the aneurysm are subjected to blood pressure and/or flow, there is a risk of the aneurysm rupturing.

Non-surgical treatments include vascular occlusion devices such as embolic coils deployed using catheter delivery systems. In a currently preferred procedure to treat a cranial aneurysm, the distal end of an embolic coil delivery catheter is initially inserted into non-cranial vasculature of a patient, typically through a femoral artery in the groin, and guided to a predetermined delivery site in a blood vessel within the cranium. The aneurysm sac is then filled with embolic material that causes formation of a solid, thrombotic mass that protects the walls from blood pressure and flow. Preferably, the thrombotic mass substantially restores the original blood vessel shape along the plane of the aneurysm's neck. The neck plane is an imaginary surface where the intima of the blood vessel would be if not for formation of the aneurysm. However, simply utilizing embolic coils is not always effective at treating aneurysms as re-canalization of the aneurysm and/or coil compaction can occur over time.

A bag for use in an aneurysm sac is described by Greenhalgh in US Patent Nos. 6,346,117 and 6,391,037, and an aneurysm neck obstruction device is shown in US Patent No. 6,454,780 by Wallace. Detachable neck bridges are disclosed by Abrams et al. in US Patent No. 6,036,720 and by Murphy et al. in US Patent No. 7,410,482 for example. Preferably, one or more embolic coils are delivered within or through the neck bridges or other obstruction devices to fill the sac of the aneurysm.

Yet another type of vaso-occlusive device is illustrated in US Patent No. 5,645,558 by Horton as having one or more strands of flexible material which are wound to form a generally spherical or ovoid vaso-occlusive structure when relaxed after being placed in a vascular cavity such as an aneurysm or fistula. Similarly, US Patent No. 5,916,235 by Guglielmi cites earlier patents describing detachable coils and then discloses an expandable cage as a vaso-occlusive structure that can receive and retain one or more coils after the cage is expanded within an aneurysm. A self-expandable aneurysm filling device is disclosed in US Patent Publication No. 2010/0069948 by Veznedaroglu et al.

It is therefore desirable to have a retrievable, repositionable device that cooperates with one or more embolic coils or other vaso-occlusive structure to effectively occlude a neck of an aneurysm or other arterio-venous malformation in a blood vessel.

### SUMMARY OF THE INVENTION

An object of the present invention is to provide an improved occlusion device which substantially blocks flow into an aneurysm in a blood vessel.

Another object of the present invention is to provide such an occlusion device which can be repositioned or retrieved from a sac of an aneurysm.

This invention features an occlusion device suitable for endovascular treatment of an aneurysm in a blood vessel in a patient, including a substantially tubular structure having a proximal end region and a distal end region, having a first, expanded condition and a second, collapsed condition. The device has dimensions in the second, collapsed condition suitable for insertion through vasculature of the patient and through a neck of the aneurysm. The device further includes a control ring having a substantially annular body disposed on the proximal end region of the structure and at least substantially circumscribing the proximal end region to prevent radial expansion of the proximal end region and to provide an engagement feature during manipulation of the occlusion device.

In a number of embodiments, the control ring defines an inner passage, such as a channel established by an inner sleeve, through which at least one embolic coil is insertable into the aneurysm. Preferably, at least a portion of the proximal end region of the tubular structure defines a plurality of openings having a sufficiently small size to enhance occlusion of the aneurysm. In some embodiments, the tubular structure cooperates with at least one vaso-occlusion structure such as a collapsible cage-like device.

In certain embodiments, the occlusive device is capable of being utilized in combination with a delivery member defining an inner lumen and having a distal end region carrying a grabber having at least two finger elements, each finger element defining a gripping region to mechanically engage the control ring. In one embodiment, the grabber is formed of a metallic material and the gripping regions are notches formed in the finger elements, each notch being sized to mechanically engage a portion of the control ring. The combination may further include a catheter having an inner lumen through which the delivery tube is insertable and translatable relative to the catheter.

This invention may also be expressed as a method of treating an aneurysm in a blood vessel in a patient, the method including selecting an occlusion device with a structure having a substantially tubular structure having a proximal end region and a distal end region, having a first, expanded condition and a second, collapsed condition, and having dimensions in the second, collapsed condition suitable for insertion through vasculature of the patient and through a neck of the aneurysm. The device further includes a control ring having a substantially annular body disposed on the proximal end region of the structure and at least substantially circumscribing the proximal end region to prevent radial expansion of the proximal end region.

In some embodiments, the method further includes mechanically engaging the control ring with a grabber on a delivery tube to enable manipulation of the occlusion device, drawing the occlusion device into a catheter carrying the delivery tube to force the occlusion device into the collapsed condition, inserting the catheter with the occlusion device into vasculature of the patient to reach the region of the aneurysm in the blood vessel, and positioning the occlusion device within the aneurysm.

In certain embodiments, the method additionally includes delivering at least one embolic coil through the delivery tube and through the control ring to secure the occlusion device within the aneurysm to occlude flow into the aneurysm, and mechanically releasing the control ring and withdrawing the catheter and the delivery tube from the patient. In yet other embodiments, the method further includes selecting the occlusive device to be attached to a collapsible cage-like vaso-occlusive structure, and positioning the occlusive device within the aneurysm includes utilizing the vaso-occlusive structure to secure the proximal end region of the tubular structure across the neck of the aneurysm.

### BRIEF DESCRIPTION OF THE DRAWINGS

In what follows, preferred embodiments of the invention are explained in more detail with reference to the drawings and photographs, in which:
FIG. 1 is a schematic side cross-sectional view of an inventive occlusion device within a novel catheter delivery system positioned at the neck of an aneurysm of a blood vessel;
FIG. 2 is an enlarged schematic side view of the delivery system of FIG. 1 showing the occlusion device held in a collapsed condition;
FIG. 3 is a schematic side view similar to FIG. 1 showing the occlusion device according to the present invention expanding within the sac of the aneurysm while still being securely held by the delivery system;
FIG. 4 is a schematic side view similar to FIG. 3 showing an embolic coil being advanced through the delivery system and the occlusion device into the aneurysm;
FIG. 5 is a schematic side view similar to FIG. 2 with the microcatheter withdrawn proximally to allow grasper fingers to release the control ring of the occlusion device;
FIG. 6 is a schematic side cross-sectional view similar to FIG. 4 after the delivery system has been withdrawn and with embolic coils securing the occlusion device within the sac of the aneurysm;
FIG. 7 is a schematic cross-sectional view of a spherical mandrel establishing the first, expanded condition for at least one an occlusion device according to the present invention;
FIGS. 8A and 8B are schematic side views of two hemi-spherical occlusion devices according to the present invention derived from the occlusion device of FIG. 7;
FIG. 9 is a schematic side view of a single occlusion device after the mandrel of FIG. 7 has been removed;
FIG. 10 is a schematic side view similar to FIG. 9 after a distal portion of the occlusion device has been removed to generate an alternative open configuration;
FIG. 11 is a side view similar to FIG. 10 of an alternative occlusion device formed utilizing an elliptical, lozenge-shaped mandrel;
FIG. 12 is a view similar to FIG. 3 showing the occlusion device cooperating with a cage-like vaso-occlusive structure within an aneurysm;
FIG. 13 is an enlarged schematic side view of an alternative delivery system for devices similar to those shown in FIG. 12 with an occlusion device and a vaso-occlusive structure held in a collapsed condition being advanced into an aneurysm;
FIG. 14 is a schematic side cross-sectional view similar to FIG. 13 after the delivery system has been withdrawn, and with the vaso-occlusive structure securing the occlusion device within the sac of the aneurysm;
FIG. 15 is a schematic side cross-sectional view of an inventive occlusion device within a novel catheter delivery system positioned at the neck of an aneurysm of a blood vessel and a catheter for delivering an embolic device inserted into the aneurysm sac;
FIG. 16 is a schematic side cross-sectional view showing the occlusion device of FIG. 15 positioned at the neck of an aneurysm of a blood vessel in a partially implanted condition and a catheter for delivering an embolic device inserted into the aneurysm sac;
FIG. 17A is a schematic side view showing the occlusion device of FIG. 16 expanded within the sac of the aneurysm while still being securely held by the delivery system and the catheter for delivering the embolic device jailed between the expanded occlusion device and a wall of the aneurysm;
FIG. 17B is a cross-sectional view of the expanded occlusion device and jailed catheter of FIG. 17A;
FIG. 18 is a schematic side view showing the expanded occlusion device of FIG. 17A and an embolic coil being advanced through the embolic delivery catheter and the occlusion device into the aneurysm;
FIG. 19 is a schematic side view showing the expanded occlusion device of FIG. 18 after completion of implantation and removal of catheters; and
FIG. 20 is a schematic side view showing the release of an expanded occlusion device such as the occlusion device illustrated in FIGs. 15-19.

### DETAILED DESCRIPTION OF THE PRESENTLY PREFERRED EMBODIMENTS

This invention may be accomplished by an occlusion device suitable for endovascular treatment of an aneurysm in a blood vessel in a patient, with a substantially tubular structure having a proximal end region and a distal end region, having a first, expanded condition and a second, collapsed condition. The device has dimensions in the second, collapsed condition suitable for insertion through vasculature of the patient, utilizing a catheter such as a microcatheter, and through a neck of the aneurysm. The device further includes a control ring having a substantially annular body disposed on the proximal end region of the structure and at least substantially circumscribing the proximal end region to prevent radial expansion of the proximal end region and to provide an engagement feature during manipulation of the occlusion device.

The control ring is releasably engageable by a releasable feature such as a grabber or at least one frangible member on a delivery member in some mechanical constructions or, in other constructions, by at least one electrolytically severable element. Preferably, the control ring defines an inner passage through which at least one embolic coil is insertable into the aneurysm. In another construction, the occlusion device is held in place within the aneurysm by at least one vaso-occlusive structure such as a cage-like device.

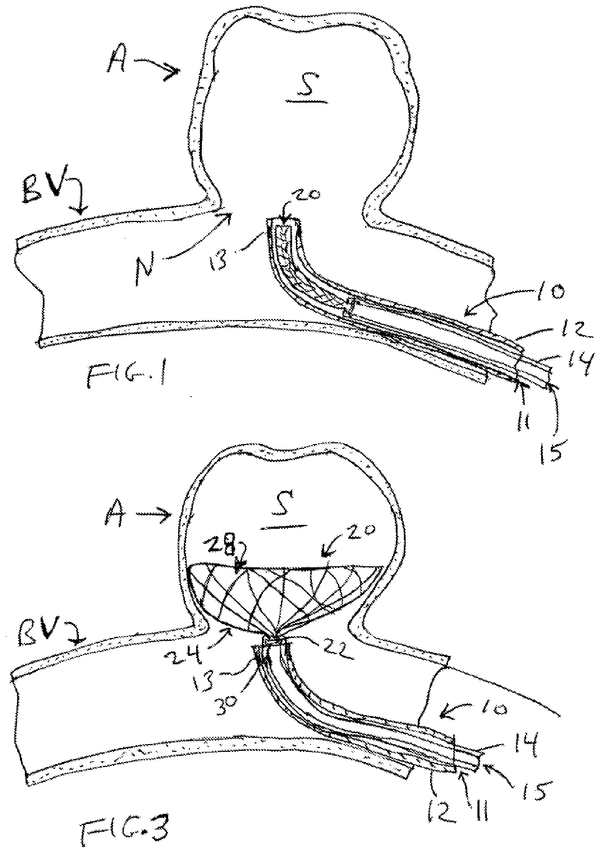

FIG. 1 schematically illustrates the distal portion of a novel delivery system 10 including a microcatheter 12 and a delivery tube 14 holding a tubular occlusion device 20 according to the present invention to be implanted within sac S of aneurysm A emerging from blood vessel BV. In one construction, the microcatheter 12 has a distal radiopaque marker band 13 and is advanced to the vicinity of neck N of aneurysm A such that marker band 13 is at the level of the neck N as seen under fluoroscopy.

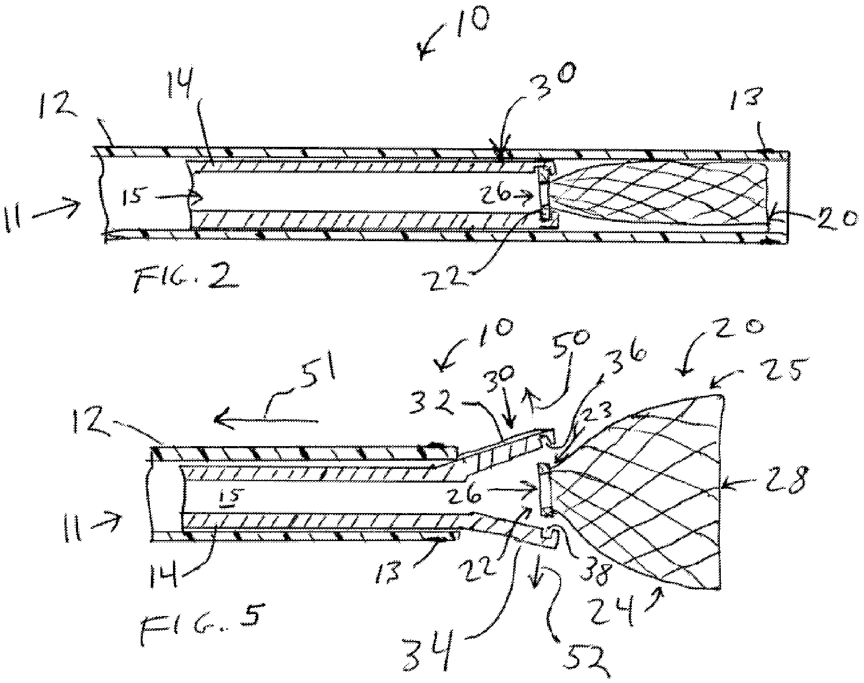

Enlarged views of the distal portion of delivery system 10 and of occlusion device 20 are provided in FIGS. 2 and 5. Occlusion device 20 is shown in a second, collapsed condition in FIG. 2 within catheter lumen 11, with a control ring 22 held by grabber 30 of delivery tube 14. Control ring 22 is disposed about a proximal region 23 of device structure 25 and defines an inner passage 26 through which one or more embolic coils are inserted, as described in more detail below. Structure 25 of occlusion device 20 further includes a mesh body 24 and a distal region 28.

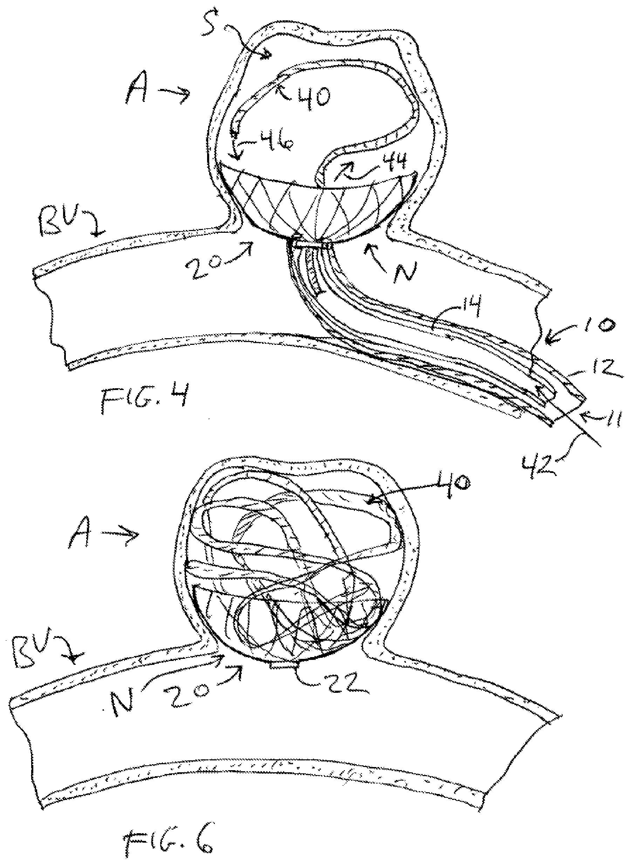

After the delivery system 10 is positioned as shown in FIG. 1, the delivery tube 14 is advanced within lumen 11 of catheter 12 to enable occlusion device 20 to expand into an approximately hemi-spherical shape within sac S as shown in FIG. 3. The shape of occlusion device 20 will conform to the shape of the sac S where device 20 touches the inner wall of the sac S. Grabber 30 continues to be constrained radially by lumen 11 of catheter 12 and maintains its grip on control ring 22 with a plurality of gripping regions such as notches 36 and 38, FIG. 5. In one construction, control ring 22 is radiopaque and is aligned under fluoroscopy relative to marker 13 on catheter 12 as shown in FIGS. 3 and 4.

Once occlusion device 20 is positioned within sac S, at least one embolic coil 40, FIG. 4, is advanced through lumen 15 of delivery tube 14 as indicated by arrow 42, through passage 26 of control ring 22 as indicated by arrow 44, and is advanced, arrow 46, within aneurysm A to substantially fill sac S and to anchor body 24 of occlusion device 20 against the interior wall of aneurysm A to block neck N as shown in FIG. 6.

After a sufficient amount of embolic coil 40 has been fully deployed within sac S to anchor occlusion device 20 within aneurysm A, the catheter 12 is withdrawn proximally, as indicated by arrow 51 in FIG. 5, while maintaining delivery tube 14 in place, to remove radial constraint on fingers 32 and 34 of grabber 30. Fingers 32 and 34 preferably are biased radially outwardly and move in the direction of arrows 50 and 52, respectively, to disengage control ring 22 from notches 36 and 38 in fingers 32 and 34, respectively.

In one construction, the catheter 12 is a polymeric microcatheter defining an inner lumen 11 having an inner diameter of between 0.020 inch and 0.027 inch, the delivery tube 14 has outer diameter that is slightly less than the inner diameter of the catheter lumen 11, and the grabber 30 with occlusion device 20 in the collapsed condition shown in FIGS. 1 and 2 also have outer diameters that are substantially the same as the inner diameter of the catheter lumen 11, which radially constrains fingers 32 and 34 to engage control ring 22. The lumen 15 of delivery tube 14 has a diameter capable of allowing passage of a conventional embolic coil delivery system having a nominal outer diameter of between 0.010 inch and 0.015 inch.

In some constructions, the delivery tube has at least one region of increased flexibility, especially near the distal end of the delivery tube, to minimize unintended microcatheter movement during translation of the delivery tube relative to the microcatheter. The at least one flexible region is made in one construction by laser-cutting a pattern of interrupted cuts into a medical-grade nitinol (NiTi) tube. In other constructions, a coiled metallic or polymeric cylindrical component and/or a cylindrical section of flexible polymeric material is added to the distal region of the delivery tube. The grabber is created in some constructions by laser-cutting material forming the grabber to create at least two finger elements, each preferably having a notch to enhance gripping of a control ring according to the present invention. In certain constructions, the grabber is integral, that is, is monolithically formed with the same material as the remainder of the delivery tube and, in other constructions, is fixedly attached to the distal end of the delivery tube.

In one construction, the structure 25 of occlusion device 20 is formed of metallic filaments that establish an expandable braided mesh tube. Suitable materials for the filaments include nitinol wires and other biocompatible metals, such as platinum, that will not remain in a collapsed condition after being ejected from a delivery tube. Preferably, at least one platinum wire is included for radiopacity. In other constructions, the structure 25 is formed of at least one polymeric material that does not become "set" in the collapsed condition.

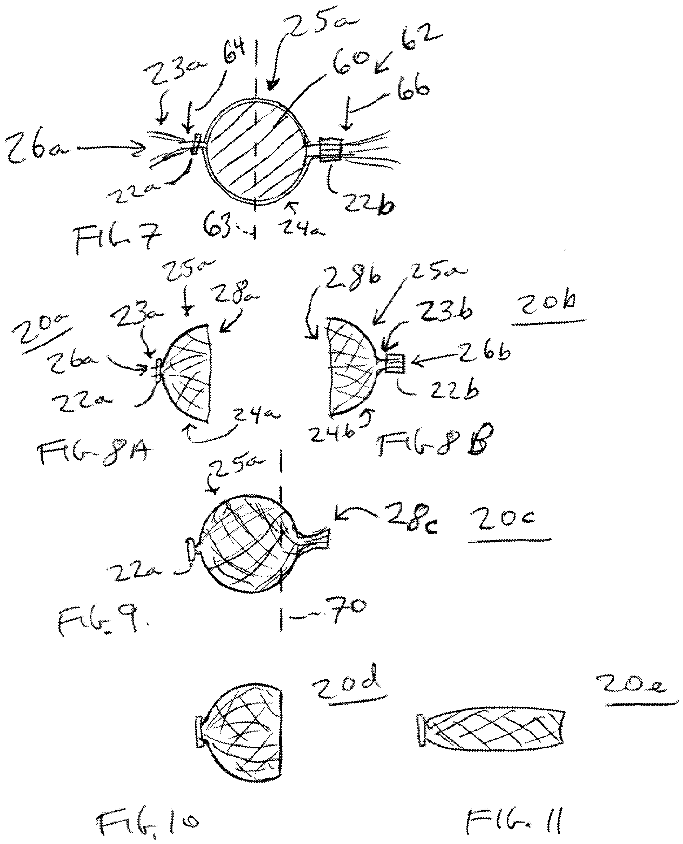

Suitable materials for control ring 22 discussed above, and for control ring 22a and band 22b discussed below in relation to FIGS. 7-8B, include biocompatible radiopaque materials such as platinum, tantalum and gold. Other suitable metallic materials include cobalt chromium, stainless steel, and combinations of two or more of biocompatible metals. Suitable polymeric materials include biocompatible biodegradable and non-biodegradable materials, as described in more detail below.

One technique for manufacturing an occlusion device according to the present invention is illustrated in FIG. 7. After structure 25a is formed as a braided mesh tube, a control ring 22a is disposed by crimping and/or welding ring material about proximal region 23a to limit radial expansion at that site while defining an inner passage 26a through which one or more embolic coils can be inserted, as described above. Optionally, an inner sleeve such as a grommet (not shown) is inserted within structure 25a and positioned under the control ring 23a to maintain an inner diameter opening of desired dimension for inner passage 26a.

In this technique, a spherical mandrel 60 such as a steel ball bearing is inserted through distal region 28a to enlarge and expand the structure 25a in body region 24a. A clamp-like element such as a band 22b is then crimped over distal region 62 to further shape the body 24a. In some techniques, the assembly is heated to set mesh body 24a in the expanded condition.

When two hemispherical occlusion devices are desired, a cut is made along the circumference of mandrel 60, typically equidistant between control ring 22a and band 22b as indicated by dashed line 63, as well as on the opposite sides of control ring 22a and band 22b as shown by arrows 64 and 66, respectively. This technique creates two separate devices 20a and 20b, as depicted in FIGS. 8A and 8B, respectively. Distal end regions 28a and 28b are both open, such as illustrated for device 20 in FIGS. 1-6. Device 20b also has body 24b, proximal region 23b, and a passage 26b formed by band 22b which serves as a control ring according to the present invention. In other words, band 22b is incorporated into an implantable device 20b in one construction, instead of being a temporary clamp.

In alternative techniques, band 22b is removed and mandrel 60, FIG. 7, is extracted to form the occlusion device 20c, FIG. 9, with a constricted yet un-constrained distal region 28c, having a single control ring 22a. In yet another technique, a cut is made non-equatorially about structure 25a, such as along line 70, to generate device 20d, FIG. 10. In yet other constructions, a non-spherical mandrel such as a lozenge-shaped mandrel, is utilized to form an elongated device 20e, FIG. 11. In other words, the occlusion device according to the present invention can have many shapes such as round, elliptic, oblong, or otherwise asymmetric, and can have an open or a closed distal end. It is expected that an open distal end will typically allow better conformance to the neck and sac of the aneurysm to be treated.

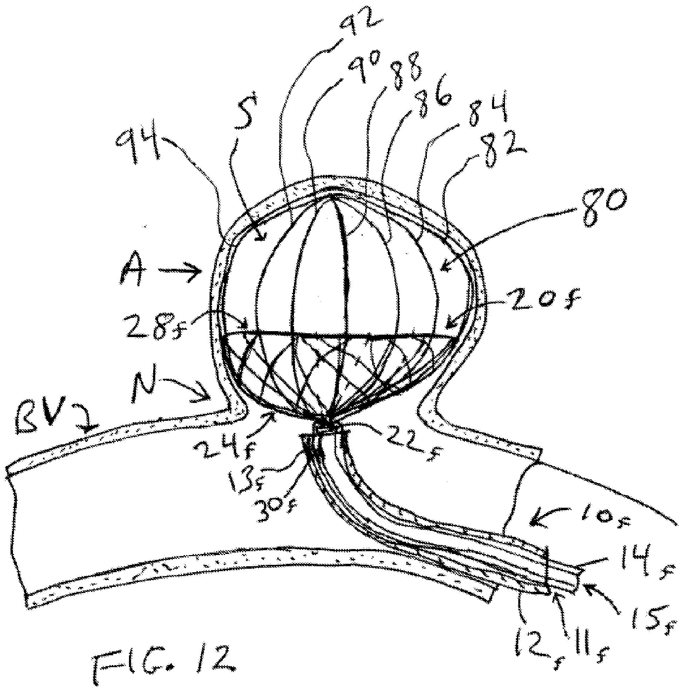

An alternative occlusion device 20f according the present invention is illustrated in FIG. 12 cooperating with a cage-like vaso-occlusive structure 80 formed of strands 82, 84, 86, 88, 90, 92 and 94 in this construction. In some constructions, vaso-occlusive structure 80 is similar to one of the embodiments disclosed in US Patent No. 5,645,558 by Horton and, in certain other constructions, is similar to one of the embodiments disclosed in US Patent No. 5,916,235 by Guglielmi and in US Patent Publication No. 2010/0069948 by Veznedaroglu et al.

After a delivery system 10f is positioned as desired relative to aneurysm A, an elongated delivery member 14f is advanced within lumen 11f of catheter 12f to enable occlusion device 20f and vaso-occlusive structure 80 to expand within sac S as shown in FIG. 12. In this construction, a grabber 30f continues to be constrained radially by lumen 11f of catheter 12f and maintains its grip on control ring 22f with a plurality of gripping regions. In one construction, control ring 22f is radiopaque and is aligned under fluoroscopy in a similar manner as described above relative to FIGS. 3 and 4.

Once vaso-occlusive structure 80 is fully deployed in an expanded condition within sac S, structure 80 presses occlusion device 20f against the interior wall and across the neck N of aneurysm A to secure it in place. In other words, vaso-occlusive structure 80 serves in an expanded condition as a frame or lattice to anchor occlusion device 20f against neck N, and occlusion device 20f, held in place by structure 80, serves as a cover extending at least across neck N, the cover preferably being porous or otherwise defining sufficiently small openings, to enhance occlusion of aneurysm A. Preferably, occlusion device 20f is secured to vaso-occlusive structure 80 by at least one attachment point, being attached to at least one of a portion of the interior surface of device 20f and a portion of the control ring 22f, to maintain an aligned relationship between the device 20f and the structure 80, especially during loading and delivery of structure 80 and device 20f utilizing a delivery cannula.

In certain techniques, if a surgeon or other user desires to substantially fill the interior of sac S, at least one embolic coil is advanced through lumen 15f of delivery tube 14f, through a passage in control ring 22f, and then is advanced into aneurysm A. In other constructions, for use where insertion of one or more embolic coils is not desired, control ring 22f may lack a passage.

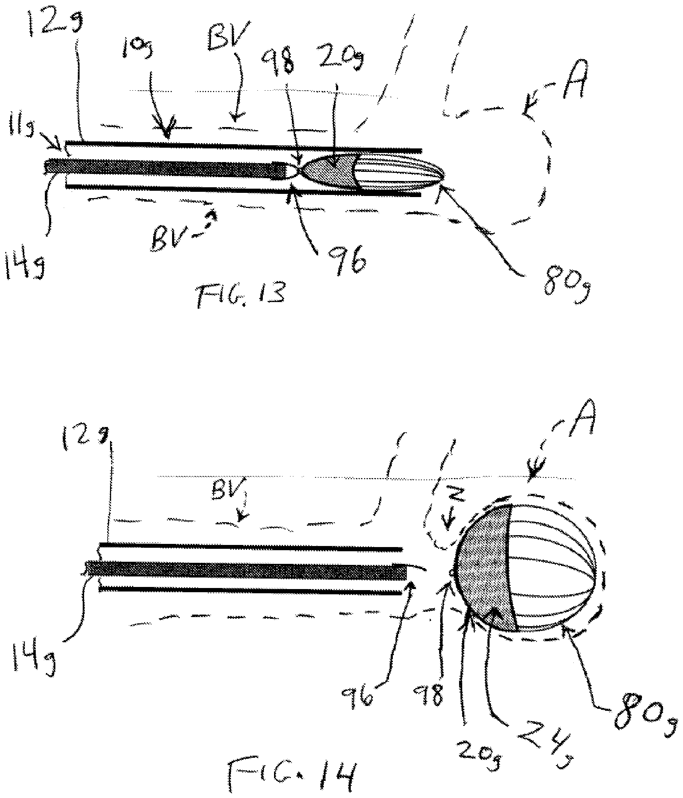

In yet other constructions, such as illustrated in FIGS. 13-14, an occlusion device 20g has a detachment feature 98, representing a conventional detachment joint, instead of a control ring. Examples of electrolytically severable joints and mechanical joints are described in US Patent No. 6,454,780 by Wallace and in US Patent No. 7,410,482 by Murphy et al., for example. Similar detachable joints are described in US Patent No. 5,916,235 by Guglielmi for cage-like vaso-occlusive structures.

After the delivery system 10g is positioned within blood vessel BV as shown in FIG. 13, a delivery member 14g, also referred to as a pusher 14g, is advanced within lumen 11 g of catheter 12g to enable occlusion device 20g and vaso-occlusive structure 80g to expand within aneurysm A as shown in FIG. 14. The connection between severable element 96 and detachment feature 98 is then severed, mechanically and/or electrolytically.

Body 24g is formed of a wire mesh or braid in some constructions. In yet other constructions, the body of the occlusive device is a biocompatible film made from one or more polymeric substances. Suitable biocompatible compositions for film material include films or matrices of cellulose, alginate, cross-linked gels, and very thin polymer films of materials such as urethane, polycaprolactone (PCL), poly-lactic acid (PLA) and/or poly-glycolic acid (PGA). The film need not be erodible or bioabsorbable. In some constructions, microscopic pores or other openings are formed in the film having average diameters which are uniform in some constructions and non-uniform in other constructions. The geometric size of the pores is substantially constant along the length of the structure in some embodiments and, in other embodiments, varies along the length. The number of pores is substantially uniform along the length of the structure in some embodiments and, in other embodiments, varies along the length. Other potential materials include polysaccharides, colloidal compounds, and some lipid products. In an alternate configuration, at least the body of the occlusive device is made of a durable, non-erodible, non-bioabsorbable material, such as a solidified urethane foam or expanded polytetrafluoroethylene (PTFE). In some embodiments, the material defines openings at least 10 microns in diameter prior to implantation in the patient and has a thickness ranging between 10 microns to 500 microns.

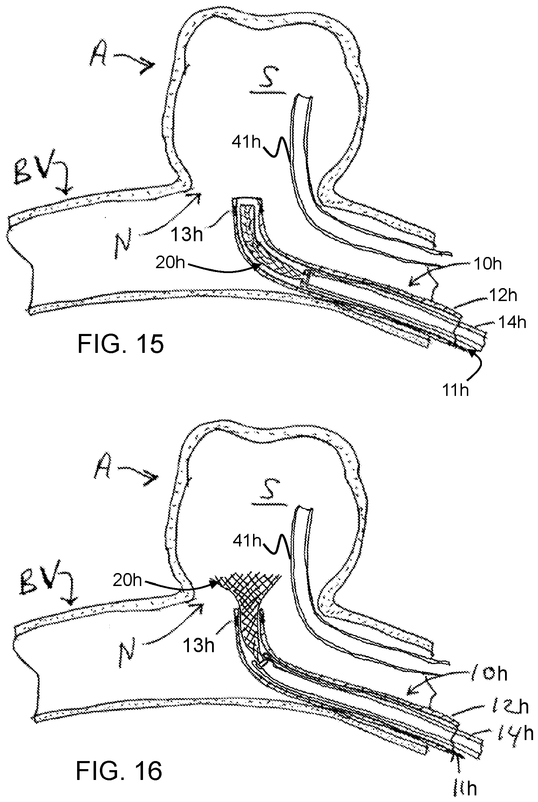

FIG. 15 schematically illustrates the distal portion of a novel delivery system 10h including an occlusion device delivery catheter 12h, a delivery tube 14h positioned within a lumen 11h of the occlusion device delivery catheter 12h holding a tubular occlusion device 20h, and an embolic implant delivery catheter 41h according to the present invention. As illustrated, the embolic implant delivery catheter 41h can be delivered to the aneurysm A separately from the occlusion device 20h. A distal end of the embolic implant delivery catheter 41h can be inserted into the sac S of the aneurysm A, and the occlusion device delivery catheter 12h can be positioned to implant the occlusion device 20h within sac S of aneurysm A. In one construction, the microcatheter 12h has a distal radiopaque marker band 13h and is advanced to the vicinity of neck N of aneurysm A such that marker band 13h is at the level of the neck N as seen under fluoroscopy.

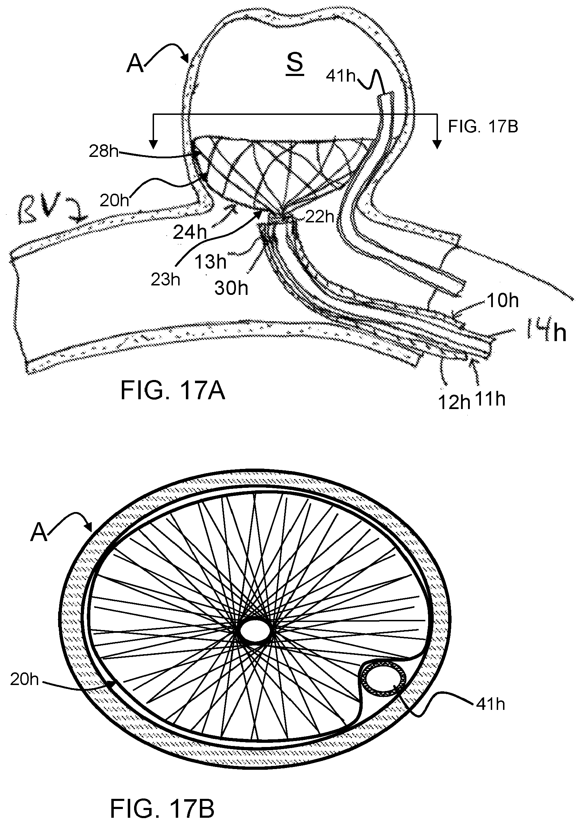

After the delivery system 10h is positioned as shown in FIG. 15, the delivery member 14h is advanced within lumen 11h of catheter 12h to enable occlusion device 20h to expand into an approximately hemi-spherical shape within sac S. FIG. 16 illustrates the expansion of a distal end region 28h of the occlusion device 20h as it exits the occlusion device delivery catheter 12h. FIG. 17A illustrates the tubular body region 24h of the occlusion device 20h expanded to such that an exterior surface of the occlusion device 20h contacts the aneurysm A and the embolic implant delivery catheter 41h. The shape of occlusion device 20h will conform to the shape of the sac S where device 20h touches the inner wall of the sac S and will conform to the embolic implant delivery catheter 41h where device 20h touches the embolic catheter 41h. FIG. 17B is a cross-sectional view of FIG. 17A illustrating the conformity of the device 20h to the inner wall of the sac S and the embolic implant delivery catheter 41h. The body region 24h of the occlusion device 20h is in the expanded condition provides a force to appose the embolic catheter 41h to the aneurysm wall.

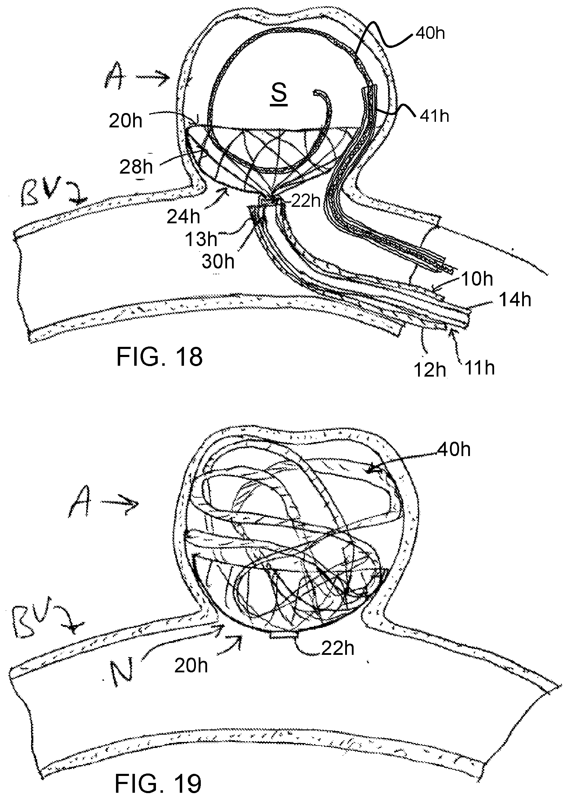

Once occlusion device 20h is positioned within sac S, at least one embolic coil 40h, FIG. 18, is advanced through a lumen of the embolic implant delivery catheter 41h to substantially fill sac S and to anchor body 24h of occlusion device 20h against the interior wall of aneurysm A to block neck N as shown in FIG. 19. As is apparent, once the embolic coil 40h is delivered, the embolic implant delivery catheter 41h can be removed. Once removed from the sac S, the occlusion device 20h can conform to the remaining section of the inner wall.

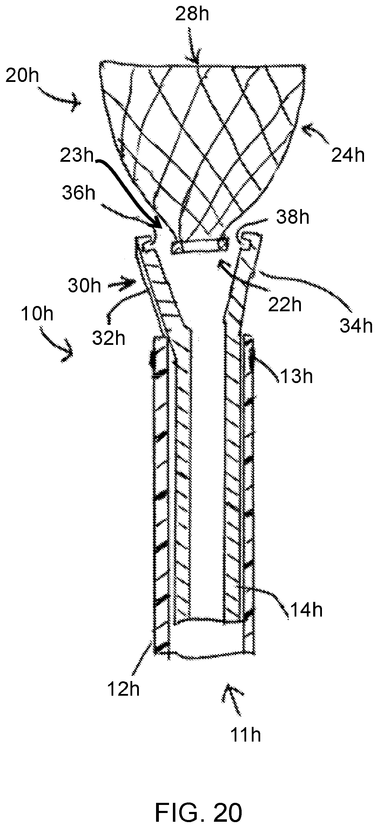

Referring collectively to FIGS. 15-18, during implantation of the occlusion device 20h and the embolic coil 40h a control ring 22h near a proximal end region 23h of the occlusion device 20h can be held by a grabber 30h on the delivery member 14h. After a sufficient amount of embolic coil 40h has been fully deployed within sac S to anchor occlusion device 20h within aneurysm A, the occlusion device 14h can be released from the delivery member 14h. The grabber 30h can be constrained by the occlusion device delivery catheter 12h, and fingers 32h and 34h of the grabber 30h can expand when exiting the occlusion device delivery catheter 12h to release the control ring 22h as shown in FIG. 20. The grabber 30h can have a plurality of gripping regions such as notches 36h and 38h. In one construction, control ring 22h is radiopaque and is aligned under fluoroscopy relative to marker 13h on catheter 12h as shown in FIGS. 15-18 and 20. Fingers 32h and 34 preferably are biased radially outwardly to disengage control ring 22h from notches 36h and 38h in fingers 32h and 34h, respectively.

An advantage of the system 10h illustrated in FIGS. 15-20 is that the occlusion device delivery catheter 12h, the control ring 22h, and the delivery member 14h need not be sized to deliver an embolic implant. Because the delivery member 14h need not be sized to delivery an embolic implant, numerous alternative delivery or pusher apparatus can be used in place of or in addition to the delivery members and delivery tubes described herein.

In one construction, the tubular structure, mesh body region 24h of occlusion device 20h is formed of metallic filaments that establish an expandable braided mesh tube. Suitable materials for the filaments include nitinol wires and other biocompatible metals, such as platinum, that will not remain in a collapsed condition after being ejected from a delivery tube. Preferably, at least one platinum wire is included for radiopacity. In other constructions, the tubular structure 24h is formed of at least one polymeric material that does not become "set" in the collapsed condition.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the spirit and scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

Every issued patent, pending patent application, publication, journal article, book or any other reference cited herein is each incorporated by reference in their entirety.

### ASPECTS OF THE INVENTION

1. A method of treating an aneurysm in a blood vessel in a patient, comprising:
   selecting an occlusion device including a substantially tubular structure having a proximal end region and a distal end region, having a first, expanded condition and a second, collapsed condition when drawn into an occlusion device delivery catheter, and further including a control ring having a substantially annular body disposed on the proximal end region of the tubular structure and at least substantially circumscribing the proximal end region to prevent radial expansion of the proximal end region, the tubular structure is a braided mesh tube, the tubular structure having a substantially hemispherical shape in the expanded condition;
   drawing the occlusion device into the occlusion device delivery catheter to force the occlusion device into the collapsed condition;
   inserting a distal end of an embolic implant delivery catheter into the aneurysm;
   inserting the occlusion device delivery catheter with the occlusion device into vasculature of the patient to reach the region of the aneurysm in the blood vessel;
   expanding the occlusion device to capture the embolic implant delivery catheter against a wall of the aneurysm;
   positioning the occlusion device within the aneurysm; and
   releasing the control ring at the proximal end region and withdrawing the occlusion device delivery catheter from the patient.
2. The method of aspect 1 wherein the tubular structure includes braided filaments.
3. The method of aspect 1 further including delivering at least one embolic coil through the embolic implant catheter to occlude flow into the aneurysm.
4. The method of aspect 3 further including providing a force by the embolic coil to secure the occlusion device within the aneurysm.
5. The method of aspect 1 further including withdrawing the embolic implant delivery catheter from the aneurysm.
6. The method of aspect 1 further including mechanically engaging the control ring with a grabber on a delivery member to enable manipulation of the occlusion device and utilizing the grabber to draw the occlusion device into the occlusion device delivery catheter carrying the delivery member to force the occlusion device into the collapsed condition.
7. A method of treating an aneurysm in a blood vessel in a patient, comprising:
   selecting an occlusion device including a substantially tubular structure having a proximal end region and a distal end region, having a first, expanded condition and a second, collapsed condition when drawn into a delivery microcatheter, at least a portion of the proximal end region defining a plurality of openings having a sufficiently small size to enhance occlusion of the aneurysm, and further including a control ring having a substantially annular body disposed on the proximal end region of the tubular structure and at least substantially circumscribing the proximal end region to prevent radial expansion of the proximal end region, the tubular structure is a braided mesh tube, the tubular structure having a substantially hemispherical shape in the expanded condition;
   mechanically engaging the control ring with a grabber on a pusher member to enable manipulation of the occlusion device;
   drawing the occlusion device into the delivery microcatheter carrying the pusher member to force the occlusion device into the collapsed condition;
   inserting the delivery microcatheter with the occlusion device into the vasculature of the patient to reach the region of the aneurysm in the blood vessel;
   inserting a distal end of an embolic implant catheter into the aneurysm;
   positioning the occlusion device within the aneurysm;
   expanding the occlusion device to capture the embolic implant catheter against a wall of the aneurysm;
   delivering at least one embolic coil through the embolic implant catheter to occlude flow into the aneurysm; and
   mechanically releasing the control ring and withdrawing the delivery microcatheter and the embolic implant catheter from the patient.
8. The method of aspect 7 further including providing a force by the embolic coil to secure the occlusion device within the aneurysm.

## Claims

1. A system suitable for endovascular treatment of an aneurysm in a blood vessel in a patient, comprising:
an occlusion device having a substantially tubular braided mesh structure having a proximal end region and a distal end region and a control ring having a substantially annular body disposed on the proximal end region of the tubular structure, the control ring at least partially circumscribing the proximal end region to prevent radial expansion of the proximal end region and to provide an engagement feature during manipulation of the occlusion device; and
an embolic implant delivery catheter capable of delivering an embolic implant to the aneurysm,
wherein the tubular structure is expandable from a collapsed condition to an expanded condition,
wherein in the collapsed condition, the tubular structure is suitable for insertion through vasculature of a patent and through a neck of the aneurysm, and
wherein in the expanded condition, the tubular structure has a substantially hemispherical shape and has an exterior surface capable of contacting the aneurysm and capable of providing a force to appose the embolic implant delivery catheter to the aneurysm.

2. The system of claim 1 further comprising the embolic implant.

3. The system of claim 2 wherein the tubular structure of the occlusion device in the expanded condition is capable of supporting the embolic implant in the aneurysm.

4. The system of claim 1 wherein the control ring is crimped onto the proximal end region of the tubular structure.

5. The system of claim 1 wherein the control ring includes radiopaque material.

6. The system of claim 1 wherein the tubular structure includes a plurality of filaments.

7. The system of claim 1 wherein the tubular structure defines a substantially enclosed volume.

8. The system of claim 1 wherein at least a portion of the proximal end region of the tubular structure defines a plurality of openings having a sufficiently small size to enhance occlusion of the aneurysm.

9. The system of claim 1 further comprising a delivery member having a distal end region carrying a grabber having at least two finger elements, each finger element defining a gripping region to mechanically engage the control ring.

10. The system of claim 9 wherein the delivery member is a tube formed of at least one metallic material.

11. The system of claim 9 wherein the grabber is formed of a metallic material and the gripping regions are notches formed in the finger elements, each notch being sized to mechanically engage a portion of the control ring.

12. The system of claim 9 further including an occlusion device delivery catheter having an inner lumen through which the delivery member is insertable and translatable relative to the occlusion device delivery catheter.
